# EUROPÄISCHE PATENTANMELDUNG

(11) **EP 0 620 439 A2**
(43) Veröffentlichungstag der Anmeldung: **19.10.1994**
(21) Anmeldenummer: 94105527.9
(22) Anmeldetag: 09.04.1994
(51) Int. Cl.: G01N 33/53, G01N 33/68

(54) **Verfahren zur Bestimmung der Bindung von Transkriptionsfaktoren an Nukleinsäuren**

(30) Priorität: 16.04.1993 DE 4312399
(71) Anmelder: BOEHRINGER MANNHEIM GMBH, D-68298 Mannheim (DE)
(72) Erfinder: Doppler, Clemens, Dr., D-82402 Seeshaupt (DE); Hinzpeter, Matthias, Dr., D-80689 München (DE); Stockinger, Hubertus, Dr., D-82377 Penzberg (DE)

(57) **Zusammenfassung**

Die Erfindung betrifft ein Verfahren zur Bestimmung der Bindung eines Transkriptionsfaktors an eine Nukleinsäure und einen für dieses Bestimmungsverfahren geeigneten Testkit.

SEQUENZPROTOKOLL

(1) ALLGEMEINE INFORMATION:

(i) ANMELDER:
(A) NAME: Boehringer Mannheim GmbH
(B) STRASSE: Sandhofer Str. 116
(C) ORT: Mannheim
(E) LAND: Deutschland
(F) POSTLEITZAHL: D-6800

(ii) ANMELDETITEL: Supershift ELISA

(iii) ANZAHL DER SEQUENZEN: 2

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 37 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 1:

AAGGGACTTT CCGCTGGGGA CTTTCCGAGC CCTCAGA 37

(2) INFORMATION ZU SEQ ID NO: 2:

(i) SEQUENZ CHARAKTERISTIKA:
(A) LÄNGE: 28 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 2:

TCGGAAAGTC CCCAGCGGAA AGTCCCTT 28

## Beschreibung

Die Erfindung betrifft ein Verfahren zur Bestimmung der Bindung eines Transkriptionsfaktors an eine Nukleinsäure und einen für dieses Bestimmungsverfahren geeigneten Testkit.

Damit die in Nukleinsäuren gespeicherte genetische Information genutzt werden kann, müssen Proteine an diese Nukleinsäuren binden. Zu diesen Proteinen zählen zunächst die RNA-Polymerasen, welche in einem als Transkription bezeichneten Vorgang eine RNA gemäß einer DNA-Vorlage synthetisieren. Zur Bindung an die informationstragenden, als Gene bezeichneten Abschnitte der DNA benötigen diese RNA-Polymerasen die Mitwirkung weiterer Moleküle, in der Regel Proteine, welche als Transkriptionsfaktoren bezeichnet werden (T. Beebee et al., Gene Structure and Transcription, IRL Press, Oxford, Washington, D.C. (1988), 73). Über die Gegenwart oder Aktivität dieser Transkriptionsfaktoren kann eine regulierte, den entsprechenden Umweltbedingungen angepaßte Nutzung der genetischen Information bewirkt werden. Eine Reihe von solchen Transkriptionsfaktoren ist inzwischen bekannt, z.B. NF_{K}B (K. Kawakami et al., Proc. Natl. Acad. Sci. USA 85 (1988), 4700 - 4704), Oct1 und Oct3 (J. Takeda et al., Nucleic Acids Research, Vol. 20 (1992), 4613 - 4620), Sp1 (G. Elder et al., Nucleic Acids Research, Vol. 20, Nr. 23 (1992), 6281 -6285; J. T. Kadonaga et al., Cell 51 (1987), 1079) sowie der Transkriptionfaktor AP-1, der als Heterodimer aus c-fos und c-jun Polypeptiden zusammengesetzt ist (D. Williams et al., Biochimica et Biophysica Acta 1180 (1992), 9 - 14; G. Schalasta et al., Molecular and Cellular Biology (1990), 5558 - 5561; D. Bohmann et al., Science 238 (1987), 1386; D. Bohmann und R. Tijan, Cell 59 (1989), 709) und AP-2 (T. Williams et al., Genes and Dev. 2 (1988), 1557 und P. J. Mitchell et al., Genes and Dev. 5 (1991), 105). Jeder dieser Transkriptionsfaktoren bindet spezifisch an eine bestimmte als Bindemotiv für diesen Transkriptionsfaktor bezeichnete DNA-Sequenz. Derartige Bindemotive sind für viele dieser Transkriptionsfaktoren bereits beschrieben worden (Biological Research Products, Promega (1992/93), 256). Diese Bindemotive liegen in der Regel upstream, also 5'-seitig zum Transkriptionsstart, können jedoch insbesondere bei den durch die RNA-3lymerase III transkribierten Genen auch im transkribierten Bereich selbst liegen.

Für die Untersuchung von Transkriptionsfaktoren und deren Bindemotiven wird eine Nukleinsäure allein bzw. in Gegenwart des entsprechenden Transkriptionsfaktors elektrophoretisch aufgetrennt. Die Bindung des Transkriptionsfaktors an die Nukleinsäure führt zu einer veränderten elektrophoretischen Beweglichkeit, an der die Bindung des Transkriptionsfaktors an die Nukleinsäure erkannt werden kann (Gel Retardation Assay, J. D. Dignam et al., Nucleic Acids Research 11 (1983), 1475). Dieser Unterschied in der elektrophoretischen Beweglichkeit kann durch vorherige Bindung eines Antikörpers an den Transkriptionsfaktor noch verstärkt werden (sog. "Super-Shift" (Y. M. Lee et al., Mol. Cell. Biol. 13 (1993), 432). Ein wesentlicher Nachteil dieses Verfahrens liegt darin, daß es aufgrund der erforderlichen elektrophoretischen Auftrennung recht zeitaufwendig und für routinemäßige Untersuchungen schwer automatisierbar ist. Zudem ist über den Nachweis im Elektrophorese-Gel nur eine qualitative Aussage möglich, ob eine Bindung des Transkriptionsfaktors an die Nukleinsäure vorliegt oder nicht.

Aufgabe der Erfindung war es daher, ein einfaches Verfahren zur Bestimmung der Bindung von Transkriptionsfaktoren an Nukleinsäuren zur Verfügung zu stellen.

Diese Aufgabe wird gelöst durch ein Verfahren zur Bestimmung der Bindung eines Transkriptionsfaktors an eine Nukleinsäure, welches dadurch gekennzeichnet ist, daß man
a) entweder die Nukleinsäure oder den Transkriptionsfaktor an eine feste Phase bindet,
b) den Transkriptionsfaktor mit der Nukleinsäure inkubiert,
c) einen markierten Antikörper, der gegen einen der beiden Bindungspartner Nukleinsäure und Transkriptionsfaktor gerichtet ist, zugibt, wobei für den Fall, daß ein markierter Antikörper gegen den gemäß a) an die feste Phase gebundenen ersten Bindungspartner verwendet wird, die Bindung des markierten Antikörpers mit dem zweiten Bindungspartner um die Bindung an den ersten Bindungspartner konkurriert,
d) feste und flüssige Phase trennt und
e) die Markierung in einer der beiden Phasen als Maß für die Bindung des Transkriptionsfaktors an die Nukleinsäure bestimmt.

Es hat sich überraschenderweise gezeigt, daß mit diesem erfindungsgemäßen Verfahren mehr Aussagen über die Bindung des Transkriptionsfaktors an die Nukleinsäure gewonnen werden können als durch einen herkömmlichen Gel-Retardation-Assay. So kann z. B. die Anzahl der Bindungsstellen auf der Nukleinsäure für den Transkriptionsfaktor bestimmt werden. Selbst Gemische langer und komplexer Nukleinsäuren können dabei ohne vorherige elektrophoretische Auftrennung eingesetzt werden, ohne daß die Bindung des Transkriptionsfaktors an seine Bindungsstelle beeinträchtigt wird. Als Nukleinsäuren können u. a. einzelsträngige wie doppelsträngige DNA als auch RNA sowie DNA-RNA-Hybride verwendet werden. Dabei spielt die Länge der Nukleinsäure keine Rolle.

Der Transkriptionsfaktor kann sowohl als eine aus biologischem Material isolierte oder rekombinant hergestellte Verbindung als auch als Rohextrakt aus biologischem Material im erfindungsgemäßen Verfahren eingesetzt werden.

Zur Durchführung des erfindungsgemäßen Verfahrens wird einer der beiden Bindungspartner Nukleinsäure oder Transkriptionsfaktor an eine feste Phase nach dem Fachmann bekannten Verfahren immobilisiert. Beispielsweise kann die Nukleinsäure an verschiedene Filtermaterialien wie z. B. Nitrocellulose oder aktivierte Nylonmembranen gebunden werden. Vorzugsweise wird die Nukleinsäure jedoch biotinyliert (z. B. am 3'- oder 5'-Ende; A. F. Cook et al., Nucleic Acids Research 16 (1988), 4077; A. Chollet et al., Nucleic Acids Research 13 (1985), 1529; L. K. Riley et al., DNA 5 (1986), 333; G. G. Schmitz et al., Anal. Biochem. 192 (1991), 222), um dann an eine mit Streptavidin beschichtete feste Phase wie z. B. Mikrotiterplatten, Reaktionsröhrchen oder kugelförmige Trägermaterialien gebunden werden zu können.

In einer bevorzugten Ausführungsform wird zunächst ein biotinyliertes Oligo- oder Polynukleotid an die mit Streptavidin beschichtete feste Phase gebunden. Die verwendeten Oligo- oder Polynukleotide können dabei sowohl chemisch synthetisiert als auch als Fragment einer natürlichen Nukleinsäure erhalten worden sein. Diese immobilisierten Oligo- oder Polynukleotide können dann als Fangprobe bei Inkubation mit einem komplexen Nukleinsäuregemisch spezifisch die Immobilisierung einer mit diesen Oligo- oder Polynukleotiden hybridisierenden Nukleinsäure bewirken. Durch diesen Anreicherungsschritt kann eine Erhöhung der Sensitivität erreicht werden.

Die Bindung des Transkriptionsfaktors an die feste Phase erfolgt ebenfalls nach dem Fachmann bekannten Verfahren, z.B. über einen an die feste Phase gebundenen Antikörper gegen den Transkriptionsfaktor oder durch Bindung eines biotinylierten Transkriptionsfaktors an eine mit Streptavidin beschichtete feste Phase.

Zum Nachweis einer Bindung zwischen den beiden Bindungspartnern Nukleinsäure und Transkriptionsfaktor wird ein markierter Antikörper, der gegen einen der beiden Bindungspartner gerichtet ist, verwendet. Diese Antikörper sind entweder selbst durch ein Enzym, Chemilumineszenz- oder Fluoreszenzfarbstoff markiert oder werden bei ihrer Verwendung im erfindungsgemäßen Verfahren durch Zugabe eines zweiten markierten Antikörpers, der gegen diesen ersten Antikörper gerichtet ist, markiert. Als Antikörper gegen den Bindungspartner Transkriptionsfaktor kann insbesondere auch ein solcher Antikörper verwendet werden, welcher gegen eine Modifikation wie z. B. eine Glykosylierung oder Phosphorylierung des Transkriptionsfaktors gerichtet ist. Da die Aktivität vieler Transkriptionsfaktoren durch die Anwesenheit bzw. Abwesenheit derartiger Modifikationen reguliert wird, können durch Verwendung derartiger Antikörper speziell nur aktivierte Transkriptionsfaktoren bestimmt werden.

Als Antikörper gegen den Bindungspartner Nukleinsäure werden z. B. bekannte Antikörper gegen DNA oder Antikörper gegen ein haptentragendes Nukleotid, wie insbesondere Digoxigenin-markiertes dUTP, verwendet. Ein derartiges Hapten-markiertes Nukleotid kann z. B. über eine in vitro Amplifikation der im erfindungsgemäßen Verfahren eingesetzten Nukleinsäure oder durch Ligation am 5'- oder 3'-Ende in diese Nukleinsäure eingebaut werden.

Bei einer ersten Ausführungsform des erfindungsgemäßen Verfahrens wird zunächst die Nukleinsäure oder der Transkriptionsfaktor als erster Bindungspartner an eine feste Phase gebunden und nach Inkubation mit dem zweiten Bindungspartner Transkriptionsfaktor bzw. Nukleinsäure ein markierter Antikörper gegen den zweiten Bindungspartner zugegeben. Nach Trennung von fester und flüssiger Phase korreliert dann die Markierung in der festen Phase direkt mit der Bindung des zweiten Bindungspartners an den an die feste Phase gebundenen ersten Bindungspartner.

Ein besonderer Gegenstand der Erfindung ist daher eine Ausführungsform des erfindungsgemäßen Verfahrens, bei welchem man zunächst den Transkriptionsfaktor an eine feste Phase bindet, den so immobilisierten Transkriptionsfaktor mit der Nukleinsäure inkubiert und dann einen markierten Antikörper gegen die Nukleinsäure zugibt.

Ein weiterer bevorzugter Gegenstand der Erfindung ist eine besondere Ausführungsform des erfindungsgemäßen Verfahrens, bei welcher man zunächst die Nukleinsäure an eine feste Phase bindet, die so immobilisierte Nukleinsäure mit dem Transkriptionsfaktor inkubiert und dann einen markierten Antikörper gegen den Transkriptionsfaktor zugibt.

In einer weiteren Ausführungsform wird zunächst die Bindungsreaktion zwischen Nukleinsäure und Transkriptionsfaktor in Lösung durchgeführt, der gebildete Komplex durch Zugabe eines markierten Antikörpers gegen einen der beiden Bindungspartner markiert und dieser markierte Komplex über den anderen Bindungspartner an eine feste Phase gebunden. Diese Bindung an die feste Phase erfolgt vorzugsweise über einen immobilisierten Antikörper, der gegen den entsprechenden Bindungspartner gerichtet ist.

In einer zweiten Variante des erfindungsgemäßen Verfahrens erfolgt der Nachweis der Bindung zwischen Nukleinsäure und Transkriptionsfaktor in einem kompetitiven Testsystem. Hierbei wird zunächst einer der beiden Bindungspartner, vorzugsweise die Nukleinsäure, an eine feste Phase gebunden und dann mit dem zweiten Bindungspartner inkubiert. Anschließend wird ein markierter Antikörper gegen den ersten Bindungspartner zugegeben, der mit dem zweiten Bindungspartner um die Bindung an den ersten Bindungspartner konkurriert. Nach Trennung von fester und flüssiger Phase ist dann die an die feste Phase gebundene Markierung umgekehrt proportional zur Bindung des zweiten Bindungspartners an den ersten Bindungspartner.

Ein bevorzugter Gegenstand der Erfindung ist eine Variante des erfindungsgemäßen Verfahrens, bei welcher man die Nukleinsäure an eine feste Phase bindet, den Transkriptionsfaktor mit der Nukleinsäure inkubiert und dann einen markierten Antikörper gegen die Nukleinsäure zugibt, der mit dem Transkriptionsfaktor um die Bindung an die Nukleinsäure konkurriert.

Durch dieses Verfahren ist es möglich, den von der Bindung des Transkriptionsfaktors abgedeckten Bereich der Nukleinsäure einzugrenzen. Hierzu wird diese Nukleinsäure, welche das Bindemotiv für den entsprechenden Transkriptionsfaktor enthält, in einer chemischen oder in vitro Synthese in Parallelen Ansätzen an verschiedenen Positionen der Nukleinsäuresequenz über den Einbau von Digoxigenin-dUTP vormarkiert. Über das erfindungsgemäße Verfahren wird dann untersucht, welche Positionen durch die Bindung des Transkriptionsfaktors sterisch abgeschirmt werden. Diejenigen Nukleinsäuren, welche die Digoxigenin-dUTP-Vormarkierung in einer solchen Position tragen, zeigen eine kompetitive Hemmung der Bindung von markiertem Antikörper gegen Digoxigenin durch den Transkriptionsfaktor. Bei Nukleinsäuren, welche außerhalb der Bindungsregion für den Transkriptionsfaktor mit Digoxigenin-dUTP vormarkiert wurden, wird dagegen keine solche kompetitive Hemmung beobachtet. Durch Verwendung von an verschiedenen Stellen vormarkierten Nukleinsäuren kann dann die Bindungsregion für den Transkriptionsfaktor eingegrenzt werden.

Vorzugsweise wird der Schritt b) des erfindungsgemäßen Verfahrens in Gegenwart einer weiteren Nukleinsäure durchgeführt, welche Bindungsstellen des Transkriptionsfaktors für repetitive Sequenzen absättigt. Repetitive Sequenzen sind Sequenzen, die in mehr als einer Kopie im Genom auftreten. Vorzugsweise wird als weitere Nukleinsäure Poly(dIdC) verwendet. Diese weitere Nukleinsäure wird dabei im molaren Überschuß (im Vergleich zur Nukleinsäure im Test) eingesetzt.

Ein weiterer Gegenstand der Erfindung ist die Verwendung des erfindungsgemäßen Verfahrens zum Auffinden weiterer Transkriptionsfaktoren, die z.B. bei Autoimmunerkrankungen wie rheumatoider Arthritis oder in der Onkologie eine Rolle spielen. Der Nachweis solcher spezifischer Transkriptionsfaktoren ist von Bedeutung für eine diagnostische Bestimmung des Anteils aktivierter Zellen oder von Genprodukten, die einen wichtigen Hinweis für das Vorliegen einer der genannten Erkrankungen geben.

Ein weiterer Gegenstand der Erfindung ist daher die Verwendung des erfindungsgemäßen Verfahrens zum Nachweis von aktivierten Zellen für einen diagnostischen Nachweis von Autoimmunerkrankungen oder Tumoren.

Ein weiterer Gegenstand der Erfindung ist schließlich ein Testkit zum Nachweis der Bindung eines Transkriptionsfaktors an eine Nukleinsäure, welcher die folgenden Bestandteile enthält: eine Nukleinsäure, welche eine Bindungsstelle für den Transkriptionsfaktor enthält sowie einen Antikörper gegen den Transkriptionsfaktor. Dabei kann der Antikörper gegen den Transkriptionsfaktor entweder selbst markiert sein oder der Testkit einen weiteren markierten Antikörper gegen den ersten Antikörper enthalten.

Vorzugsweise ist die Nukleinsäure biotinyliert und der Testkit enthält zusätzlich eine mit Streptavidin beschichtete feste Phase zur Immobilisierung der biotinylierten Nukleinsäure.

Besonders bevorzugt enthält dieser Testkit eine Nukleinsäure mit dem Bindemotiv für den Transkriptionsfaktor NF_{K}B (K. Kawakami et al., Proc. Natl. Acad. Sci. USA 85 (1988), 4700 - 4704). Ganz besonders bevorzugt enthält der Testkit eine Nukleinsäure mit der in SEQ ID NO 1 und/oder 2 gezeigten Sequenz, die natürlich in einer für den Fachmann geläufigen Weise variiert werden kann, solange die Bindungseigenschaften für den Transkriptionsfaktor NF_{K}B erhalten bleiben.

Die Erfindung wird durch das folgende Beispiel zusammen mit den Sequenzprotokollen näher erläutert.
- SEQ ID NO 1 und 2: zeigt die Sequenz des Bindemotivs für den Transkriptionsfaktor NF_{K}B, wobei SEQ ID NO 1 und 2 zusammen eine doppelsträngige DNA ergeben.

### Beispiel 1

### Nachweis der Bindung eines Kernextraktes aus aktivierten Jurkat-Zellen an DNA

Jurkat-Zellen (ATCC TIB 152) werden für 8 h mit 10 nmol/l PMA (Tetraphorbolmyristatacetat, W. Phares et al., J. Virol. 66 (1992), 7490) und 2 µg/ml PHA (Phytohämaglutinin) stimuliert. Anschließend wird der Kernextrakt nach Standardverfahren (z. B. gemäß J. D. Dignam et al., Nucleic Acids Research 11 (1983), 1475) hergestellt und bei -80°C aufbewahrt.

Um eine Oligonukleotid-DNA mit dem Bindemotiv für den Transkriptionsfaktor NF_{K}B zu erhalten, werden die in SEQ ID NO 1 und 2 gezeigten Oligonukleotide auf chemischem Wege nach bekannten Verfahren über eine Festphasensynthese hergestellt. Die beiden erhaltenen Oligonukleotide bilden zusammen eine doppelsträngige DNA mit dem Bindemotiv für den Transkriptionsfaktor NF_{K}B aus.

Für die Bindereaktion werden 1 - 5 µg Kernextraktprotein (Bestimmung nach M. M. Bradford, Anal. Biochem. 72 (1976), 248) in 2 µl Inkubationspuffer verdünnt und bei Raumtemperatur mit 7 µl Bindepuffer und 1 µl biotinylierter Oligonukleotid-DNA (10⁻¹² mol, Bestimmung gemäß J. Sambrook et al., Molecular Cloning, A Laboratory Manual, Gold Spring Harbour Laboratory Press (1989), Biotinylierung gemäß L. K. Riley et al., DNA 5 (1986), 333) für 30 min inkubiert. Anschließend werden 25 µl eines Digoxigenin-markierten Antikörpers gegen den Transkriptionsfaktor NF_{K}B zu diesem Reaktionsgemisch hinzugegeben und weitere 15 - 60 min bei Raumtemperatur inkubiert. (Ein Antiserum aus der Ziege gegen NF_{K}B wurde erhalten durch Immunisierung von 5 Ziegen mit dem von Bäuerle et al. beschriebenen NF_{K}B Immunogen (EMBO J. 12 (1993), 201) und Markierung der erhaltenen polyklonalen Antikörper mit Digoxigenen gemäß Schmitz et al. (Anal. Biochem. 192 (1991), 222). Der Nachweis erfolgte durch einen POD markierten anti Digoxigenin spezifischen monoklonalen Antikörper (Boehringer Mannheim GmbH Ident. Nr. 1 333 062 001). Monoklonale Antikörper aus der Maus wurden gewonnen durch Immunisierung von 5 Mäusen mit dem oben beschriebenen Immunogen von Bäuerle et al. nach bekanntem Verfahren und mit Digoxigenin konjugiert nach Schmitz et al. Der Nachweis erfolgte mit einem POD markierten anti Digoxigenin spezifischen Antikörper (Boehringer Mannheim GmbH Ident. Nr. 1 333 062 001).) Schließlich werden 25 µl eines mit Meerrettich-Peroxidase konjugierten Antikörpers gegen Digoxigenin (Boehringer Mannheim GmbH, Katalog-Nr. 1 333 062 001) hinzugegeben, weitere 15 min bei Raumtemperatur inkubiert und anschließend 50 µl dieses Reaktionsansatzes in eine mit Streptavidin beschichtete Mikrotiterplatte (Herstellung wie in EP-A 0 269 092 beschrieben) überführt. Nach 2- bis 4-maligem Waschen mit Waschpuffer (B. Porstmann et al., J. Clin. Chem. Clin. Biochem. 19 (1981), 435) wird ABTS 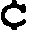 (Boehringer Mannheim GmbH, Katalog-Nr. 756407 und 1204530) als Substrat hinzugegeben und die Absorption bei 405 nm in einem ELISA-Reader bestimmt. Der Vergleich mit einem Parallelansatz, der in Gegenwart von 10⁻¹⁰ mol nicht mit Biotin markierter Oligonukleotid-DNA durchgeführt wurde, zeigt, daß spezifisch die Bindung des Transkriptionsfaktors NF_{K}B an die verwendete DNA bestimmt wird (siehe Tabelle 1).

**Tabelle 1**

| Kernextrakt (µg/ml) | Absorption (405 nm) | |
|---|---|---|
| | in Gegenwart von 10⁻¹⁰ mol nicht biotinylierter DNA | in Abwesenheit nicht biotinylierter DNA |
| 0,0 | 0,04 | 0,04 |
| 1,5 | 0,05 | 0,10 |
| 3,0 | 0,08 | 0,26 |
| 4,5 | 0,11 | 0,47 |
| 6,0 | 0,13 | 0,72 |
| 7,5 | 0,15 | 0,80 |
| 9,0 | 0,18 | 0,84 |

- Bindepuffer:: 10 mmol/l Tris-HCl (pH 7,5)
55 mmol/l Natriumchlorid
0,21 % NP-40
2,0 % Ficoll 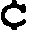 400
1,0 µg poly dI.dC
- Inkubationspuffer: 20 mmol/l Hepes (pH 7,9)
20 % (v/v) Glycerin
0,1 mol/l Kaliumchlorid
0,2 mmol/l EDTA
0,5 mmol/l PMSF (Phenylmethylsulfonylfluorid)
0,5 mmol/l DTT (Dithiothreitol)
(PMSF und DTT werden jeweils erst vor Versuchsbeginn zugegeben)
- Extraktionspuffer:: 20 mmol/l Hepes (pH 7,9)
25 % (v/v) Glycerin
420 mmol/l Natriumchlorid
1,5 mmol/l MgCl₂
0,2 mmol/l EDTA
0,5 mmol/l PMSF
0,5 mmol/l DTT

### Beispiel 2

### Nachweis der DNA-Bindung eines Kernextraktes durch Immobilisierung des gebildeten Komplexes und Nachweis über einen Anti-DNA-Antikörper

Zunächst wird aus Raji-Zellen (ATCC CCL 86) gemäß dem in Beispiel 1 beschriebenen Verfahren ein Kernextrakt hergestellt. Dieser Kernextrakt wird gemäß den Angaben in Beispiel 1 mit 10⁻¹² mol einer Oligonukleotid-DNA mit dem Bindemotiv für den Transkriptionsfaktor NF_{K}B sowie zusätzlich mit einem biotinylierten monoklonalen Antikörper gegen NF_{K}B (Herstellung analog dem in Beispiel 1 angegebenen Verfahren) inkubiert. Der gebildete Komplex wird in eine mit Streptavidin beschichtete Mikrotiterplatte überführt und nach 30minütiger Inkubation 2 mal gewaschen. Anschließend wird der immobilisierte Komplex durch Zugabe eines Peroxidase-markierten monoklonalen Antikörpers gegen DNA (Boehringer Mannheim GmbH Hr. 1525751) sowie des Substrats ABTS 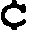 (Boehringer Mannheim GmbH, Katalog-Nr. 756407 und 1204530) und Messung der Absorption bei 405 nm in einem ELISA-Reader nachgewiesen. Die nachfolgende Tabelle 2 zeigt die Korrelation zwischen der gemessenen Markierung über die DNA und der zur Immobilisierung eingesetzten Menge an Kernextrakt.

**Tabelle 2**

| Kernextrakt in µg/ml | Absorption (405 nm) |
|---|---|
| 0,0 | 0,00 |
| 2,5 | 0,15 |
| 5,0 | 0,28 |
| 7,5 | 0,50 |
| 10,0 | 0,61 |
| 12,5 | 0,79 |
| 15,0 | 1,0 |

## Patentansprüche

1. Verfahren zur Bestimmung der Bindung eines Transkriptionsfaktors an eine Nukleinsäure, dadurch gekennzeichnet, daß man
a) entweder die Nukleinsäure oder den Transkriptionsfaktor an eine feste Phase bindet,
b) den Transkriptionsfaktor mit der Nukleinsäure inkubiert,
c) einen markierten Antikörper, der gegen einen der beiden Bindungspartner Nukleinsäure oder Transkriptionsfaktor gerichtet ist, zugibt, wobei für den Fall, daß ein markierter Antikörper gegen den gemäß a) an die feste Phase gebundenen ersten Bindungspartner verwendet wird, die Bindung des markierten Antikörpers mit dem zweiten Bindungspartner um die Bindung an den ersten Bindungspartner konkurriert,
d) feste und flüssige Phase trennt und
e) die Markierung in einer der beiden Phasen als Maß für die Bindung des Transkriptionsfaktors an die Nukleinsäure bestimmt.

2. Verfahren gemäß Anspruch 1, dadurch gekennzeichnet, daß man den Transkriptionsfaktor an eine feste Phase bindet, den Transkriptionsfaktor mit der Nukleinsäure inkubiert und einen markierten Antikörper gegen die Nukleinsäure zugibt.

3. Verfahren gemäß Anspruch 1, dadurch gekennzeichnet, daß man die Nukleinsäure an eine feste Phase bindet, die Nukleinsäure mit dem Transkriptionsfaktor inkubiert und einen markierten Antikörper gegen den Transkriptionsfaktor zugibt.

4. Verfahren gemäß einem der Ansprüche 1 oder 3, dadurch gekennzeichnet, daß der markierte Antikörper gegen eine Modifikation des Transkriptionsfaktors gerichtet ist.

5. Verfahren gemäß Anspruch 1, dadurch gekennzeichnet, daß man die Nukleinsäure an eine feste Phase bindet, die Nukleinsäure mit dem Transkriptionsfaktor inkubiert und einen markierten Antikörper gegen die Nukleinsäure zugibt, der mit dem Transkriptionsfaktor um die Bindung an die Nukleinsäure konkurriert.

6. Verfahren gemäß einem der Ansprüche 1 oder 3 bis 5, dadurch gekennzeichnet, daß man die Nukleinsäure zur Bindung an die feste Phase biotinyliert und eine mit Streptavidin beschichtete feste Phase verwendet wird.

7. Verfahren gemäß einem der Ansprüche 1 bis 6, dadurch gekennzeichnet, daß die Inkubation der Nukleinsäure mit dem Transkriptionsfaktor in Gegenwart einer weiteren Nukleinsäure durchgeführt wird, welche Bindungsstellen des Transkriptionsfaktors für repetitive Sequenzen der Nukleinsäure absättigt.

8. Verwendung eines Verfahrens gemäß einem der Ansprüche 1 bis 7 zum Nachweis von aktivierten Zellen für einen diagnostischen Nachweis von Autoimmunerkrankungen oder Tumoren.

9. Kit zum Nachweis der Bindung eines Transkriptionsfaktors an eine Nukleinsäure, enthaltend eine Nukleinsäure, welche eine Bindungsstelle für diesen Transkriptionsfaktor enthält, sowie einen Antikörper gegen diesen Transkriptionsfaktor.

10. Kit gemäß Anspruch 9, dadurch gekennzeichnet, daß die Nukleinsäure biotinyliert ist und der Kit zusätzlich eine mit Streptavidin beschichtete feste Phase zur Immobilisierung der biotinylierten Nukleinsäure enthält.

11. Kit gemäß einem der Ansprüche 9 oder 10, dadurch gekennzeichnet, daß er eine Nukleinsäure mit dem Bindemotiv für den Transkriptionsfaktor NF_{K}B enthält.

12. Kit gemäß Anspruch 11, dadurch gekennzeichnet, daß die Nukleinsäure mit dem Bindemotiv für den Transkriptionsfaktor NF_{K}B die in SEQ ID NO 1 und/oder SEQ ID NO 2 gezeigte Sequenz aufweist.
